(19) **Europäisches Patentamt / European Patent Office / Office européen des brevets**

(11) **EP 4 056 989 B1**

(12) **EUROPEAN PATENT SPECIFICATION**

(45) Date of publication and mention of the grant of the patent:
**07.06.2023 Bulletin 2023/23**

(21) Application number: **22160931.6**

(22) Date of filing: **08.03.2022**

(51) International Patent Classification (IPC):
**G01N 21/43** $^{(2006.01)}$

(52) Cooperative Patent Classification (CPC):
**G01N 21/43;** G01N 2201/0216

(54) **APPARATUS AND METHOD FOR MEASURING SALINITY**

GERÄT UND VERFAHREN ZUR SALINITÄTSMESSUNG

APPAREIL ET PROCÉDÉ DE MESURE DE SALINITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2021 JP 2021038808**

(43) Date of publication of application:
**14.09.2022 Bulletin 2022/37**

(73) Proprietor: **Yamada Giken Co., Ltd.**
**Fukui City, Fukui 918-8015 (JP)**

(72) Inventors:
• **MIKAMI, Naoto**
**Tokyo (JP)**
• **SASAKI, Hideo**
**Tokyo (JP)**
• **NAKANO, Yukihiro**
**Tokyo (JP)**
• **SUYA, Yutaka**
**Fukui City (JP)**
• **YAMADA, Takeo**
**Fukui City (JP)**
• **NAKAYAMA, Takayuki**
**Fukui City (JP)**
• **TOKUNAGA, Toru**
**Fukui City (JP)**

(74) Representative: **Vossius & Partner**
**Patentanwälte Rechtsanwälte mbB**
**Siebertstraße 3**
**81675 München (DE)**

(56) References cited:
**EP-A1- 1 602 913      DE-A1- 3 019 341**
**JP-A- 2002 236 098**

## Description

TECHNICAL FIELD

The present invention is related to an apparatus and method for measuring salinity, especially, on a road surface on which a vehicle travels, and a system for managing the salinity.

BACKGROUND ART

EP 1602913 A1 discloses a method including: sprinkling water toward a dry road surface to dissolve salinity on the road surface; bouncing up the water containing the dissolved salinity by a wheel of a vehicle; and measuring salinity concentration of the bounced water to measure salinity on the road surface.

SUMMARY OF INVENTION

Technical Problem

In the method disclosed in EP 1602913 A1, a relatively large amount of water is sprinkled in order to bounce up a large amount of water enough to sense salinity concentration. This reduces accuracy of the salinity measurement. Also, this may cause the sprinkled water to freeze.

To prevent this, EP 1602913 A1 discloses sprinkling salt water in a case that the atmospheric temperature is low. However, accuracy of the salinity measurement is further reduced in a case that the concentration of the sprinkled salt water is not adequately managed.

Solution to Problem

The invention provides an apparatus according to claim 1 for measuring salinity on a road surface. The apparatus is mounted in a vehicle having a wheel. The vehicle travels on the road surface. The vehicle is an automobile, for example. The apparatus includes the following components.

(1) A concentration sensor. The concentration sensor is disposed behind the wheel to be watered by water bounced up by the wheel. The wheel is a tire, for example. The wheel may be a drive wheel, or may be a non-drive wheel. The concentration sensor senses salinity concentration of the water bounced up by the wheel. The concentration sensor may be one disclosed in EP 1602913 A1, or may have other configurations.

(2) A front nozzle. The front nozzle is disposed ahead the wheel. The front nozzle sprinkles water toward the road surface, especially toward an area on which the wheel is to pass, to dissolve salinity on the road surface in the sprinkled water. The front nozzle sprinkles a minimum amount of water required for dissolving the salinity on the road surface to prevent reduction of accuracy of salinity measurement. Also, this enables to prevent the sprinkled water from freezing. Thus, there is no need to sprinkle salt water. This improves accuracy of salinity measurement.

(3) A rear nozzle. The rear nozzle is disposed between the wheel and the front nozzle. The rear nozzle sprinkles water toward the wheel to wet the wheel. The water attached to the wheel is mixed with the water on the road surface. Thereby, an amount of the water bounced up by the wheel becomes large enough to sense salinity concentration by the concentration sensor.

The apparatus may include an electric control unit. The electric control unit may be a computer, for example. The electric control unit may compute an amount of the salinity on the road surface. The computation may be performed based on the salinity concentration sensed by the concentration sensor and an amount of the water sprinkled by the front nozzle and the rear nozzle.

A system is for managing salinity on a road surface. The system includes the following components.

(1) The above-described apparatus.

(2) A display. The display displays a dangerous area on a map. The dangerous area is determined that an amount of salinity in the dangerous area is less than a predetermined threshold. The determination is performed based on the amount of the salinity measured by the apparatus and a position of the vehicle at a time when the concentration sensor senses the salinity concentration. The predetermined threshold is determined by considering an amount of salinity required to prevent the road surface from freezing to assure safety.

The invention further provides a method according to claim 4 for measuring salinity on a road surface. A vehicle has a wheel, and travels on the road surface. The method includes the following steps.

(1) Water is sprinkled from a front nozzle. The front nozzle is disposed ahead the wheel. The water is sprinkled toward the road surface, especially, an area on which the wheel is to pass, to dissolve salinity on the road surface in the sprinkled water. A minimum amount of water required for dissolving the salinity on the road surface is sprinkled to prevent reduction of accuracy of salinity measurement. Also, this enables to prevent the sprinkled water from freezing. Thus, there is no need to sprinkle salt water. This improves accuracy of salinity measurement.

(2) Water is sprinkled from a rear nozzle. The rear nozzle is disposed between the wheel and the front nozzle. The water is sprinkled toward the wheel to wet the wheel. The water attached to the wheel is mixed with the water on the road surface. Thereby, an amount of the water bounced up by the wheel becomes large enough to sense salinity concentration by the concentration sensor.

(3) Salinity concentration of water bounced up by the wheel is sensed by a concentration sensor. The concentration sensor is disposed behind the wheel to be watered by water bounced up by the wheel. The wheel is a tire, for example. The wheel may be a drive wheel, or may be a non-drive wheel. The concentration sensor may be one disclosed in EP 1602913 A1, or may have other configurations.

The method may include a step for computing an amount of the salinity. The computation may be performed based on the salinity concentration measured by the concentration sensor and an amount of the water sprinkled by the front nozzle and the rear nozzle.

The method may include the following steps.

(1) A position of the vehicle is sensed at a time when the concentration sensor measures the salinity concentration.

(2) A dangerous area in which the amount of the salinity is less than a predetermined threshold is determined. The determination may be performed based on the computed amount of the salinity and the measured position. The predetermined threshold may be determined by considering an amount of salinity required to prevent the road surface from freezing to assure safety.

The method may include a step for displaying the determined dangerous area on a map.

Advantageous Effects of the Invention

The wheel is wet by the water sprinkled from the rear nozzle. Thereby, even though an amount of the water sprinkled from the front nozzle toward the road surface is small, an amount of the water bounced up by the wheel is large enough to sense salinity concentration by the concentration sensor. Since the amount of the water sprinkled toward the road surface can be small, an amount of the salinity on the road surface can be precisely measured. Also, since the amount of the water sprinkled toward the road surface can be small, there is no fear that the sprinkled water freezes. Thus, there is no need to sprinkle salt water, and thereby, an amount of the salinity on the road surface can be precisely measured.

BRIEF DESCRIPTION OF DRAWINGS

FIG. 1 is a block diagram showing an example of a system according to the present invention; and
FIG. 2 is a flowchart showing an example of a method according to the present invention.

DESCRIPTION OF EMBODIMENT

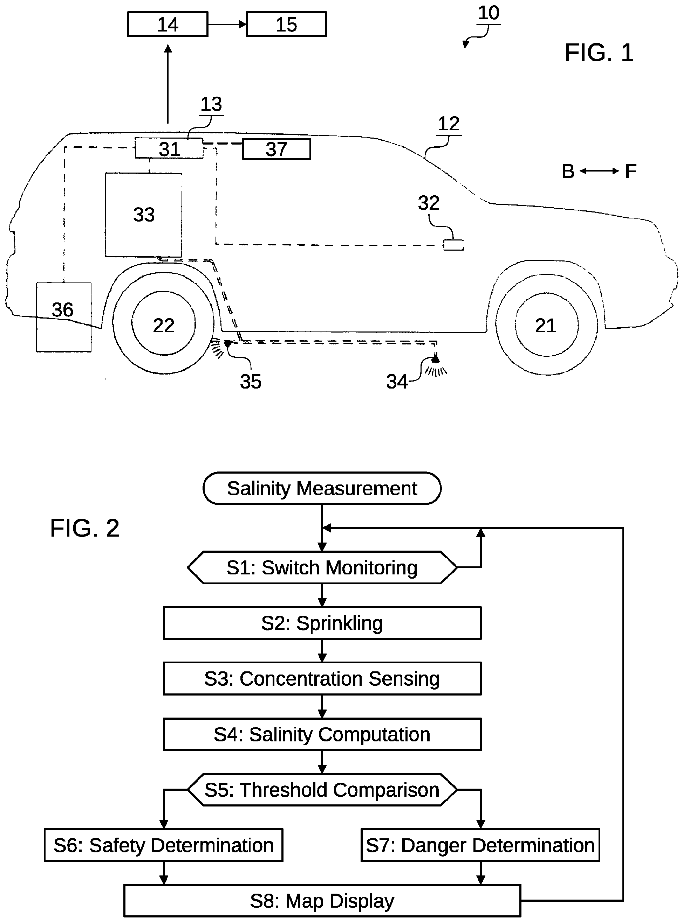

Referring FIG. 1, a salinity management system **10** measures salinity on a dry road surface. The salinity management system **10** includes a vehicle **12,** a salinity measurement apparatus **13,** a server computer **14,** and a liquid crystal display unit **15,** for example.

The vehicle **12** travels on the road surface. The vehicle **12** normally moves toward **F** direction, but it can also move toward **B** direction. The vehicle **12** is self-propelled, but it may be trailed by a self-propelled vehicle.

The vehicle **12** has two front tires **21** and two rear tires **22,** for example. The tires **21, 22** roll on the road surface.

The salinity measurement apparatus **13** is mounted in the vehicle **12.** The salinity measurement apparatus **13** measures salinity on the road surface. The salinity measurement apparatus **13** includes an electric control unit (ECU) **31,** a switch **32,** a water tank **33,** a front nozzle **34,** a rear nozzle **35,** a concentration sensor **36,** and a positioning unit **37,** for example.

The ECU **31** is a computer or other electric circuits, for example. The ECU **31** is installed in the vehicle **12.** The ECU **31** controls sprinkling and measurement.

The switch **32** is installed at an assistant driver's seat in the vehicle **12,** for example. The switch **32** is a toggle switch, for example. When the switch **32** is turned on, the sprinkling is started. When the switch **32** is turned off, the sprinkling is stopped. The switch **32** may be an automatic return switch which is automatically turned off when a predetermined time passes after the switch is turned on. Or, the switch 32 may be a push switch which is on only while the switch is pushed.

For example, the ECU **31** monitors a state of the switch **32.** When detecting that the switch **32** is on, the ECU **31** issues a sprinkling command. When detecting that the switch **32** is off, the ECU **31** stops issuing the sprinkling command. In a case that the switch **32** is a push switch, the ECU **31** may continue to issue the sprinkling command until a predetermined time passes after the switch **32** is turned on.

The water tank **33** is installed in the vehicle **12,** especially in a luggage space, for example. The water tank **33** stores water to be sprinkled. The water stored in the water tank **33** is fresh water containing no salt.

The front nozzle **34** is installed under a chassis in the vehicle **12,** for example. The front nozzle **34** communicates with the water tank **33** to sprinkle the water stored in the water tank **33.** The front nozzle **34** is directed downward to sprinkle the water toward the road surface. The front nozzle **34** is directed to sprinkle the water toward a direction perpendicular to the road surface, for example.

The front nozzle **34** is disposed at a position in **F** direction from one of the rear tires **22.** Hereinafter, the one of the rear tires **22** is called "measurement wheel."

When the vehicle **12** travels on the road surface toward **F** direction, the measurement wheel **22** passes on an area onto which the front nozzle **34** sprinkles the water.

When the switch **32** is on, the front nozzle **34** sprinkles the water. When the switch **32** is off, the front nozzle **34** stops sprinkling the water. For example, the ECU **31** issues the sprinkling command, a valve disposed at a middle of a pipe communicating between the water tank **33** and the front nozzle **34** is opened to supply water to the front nozzle **34**. When the ECU **31** stops issuing the sprinkling command, the valve is closed.

The rear nozzle **35** is installed under the chassis in the vehicle 12, for example. The rear nozzle **35** communicates with the water tank **33** to sprinkle the water stored in the water tank **33**. The rear nozzle **35** is disposed at a position between the measurement wheel **22** and the front nozzle **34**. The rear nozzle **35** is directed to the measurement wheel **22** to sprinkle the water toward the measurement wheel **22**. The rear nozzle **35** is directed to sprinkle the water toward a direction downwardly diagonal to a surface of the measurement wheel **22,** for example.

A surface of the measurement wheel **22,** especially, an area at a front side, is wetted by the water sprinkled from the rear nozzle **35**. When the vehicle **12** travels on the road surface toward **F** direction, the measurement wheel **22** rolls, and thereby, the area wet by the water sprinkled from the rear nozzle **35** touches the road surface.

When the switch **32** is on, the rear nozzle **35** sprinkles the water. When the switch **32** is off, the rear nozzle **35** stops sprinkling the water. For example, the ECU **31** issues the sprinkling command, a valve disposed at a middle of a pipe communicating between the water tank **33** and the rear nozzle **35** is opened to supply water to the rear nozzle **35**. When the ECU **31** stops issuing the sprinkling command, the valve is closed.

The concentration sensor **36** is installed under the chassis in the vehicle **12,** for example. The concentration sensor **36** senses salinity concentration of water bounced up by the measurement wheel **22**. The concentration sensor **36** is disposed at a position in **B** direction from the measurement wheel **22** to be watered by water bounced up by the measurement wheel **22.**

Since the measurement wheel **22** is wet by the water sprinkled from the rear nozzle **35** in advance, a large amount of the water enough to sense salinity concentration by the concentration sensor **36** is bounced up even when an amount of the water sprinkled from the front nozzle **34** is small.

This enables to reduce an amount of the water sprinkled from the front nozzle **34** toward the road surface. This enables to improve accuracy of the salinity measurement. Also, this enables to eliminate fear that the water sprinkled toward the road surface freezes. So, there is no need to sprinkle salt water. Since fresh water is sprinkled toward the road surface, there is no need to correct the measured salinity concentration by subtracting salinity contained in the sprinkled water. This enables to improve accuracy of the salinity measurement.

The concentration sensor **36** senses the salinity concentration once a second, for example. The concentration sensor **36** sends data representing the sensed salinity concentration to the ECU **31**.

The ECU **31** receives the data sent from the concentration sensor **36**. The ECU **31** computes an amount of salinity on the road surface based on the salinity concentration sensed by the concentration sensor **36** and an amount of the water sprinkled from the front nozzle **34** and the rear nozzle **35**. The amount of the water sprinkled from the front nozzle **34** and the rear nozzle **35** may be fixed.

For example, salinity concentration **Ci** of the water on the road surface can be represented by the following formula:

$$C_1 = R \cdot S / V,$$

where **R** denotes an amount of residual salinity per unit area on the road surface, **V** denotes a volume of the water per unit time sprinkled from the front nozzle **34,** and **S** denotes an area per unit time on which the water is sprinkled from the front nozzle **34**.

And, the salinity concentration $C_2$ of the water bounced up by the measurement wheel **22** can be represented by the following formula:

$$C_2 = p \cdot C_1,$$

where $p$ denotes a proportion of the water on the road surface within the water bounced up by the measurement wheel **22**. It should be noted that the water bounced up by the measurement wheel **22** contains the water sprinkled from the rear nozzle **35**.

Accordingly, the ECU **31** can compute the amount of residual salinity per unit area on the road surface by using the following formula:

$$R = k \cdot C_2,$$

where **k** denotes a predetermined constant, **k = V / (p·S).**

The positioning unit **37** is installed in the vehicle **12**. The positioning unit **37** measures a position of the vehicle **12**. The positioning unit **37** may be a receiver of the Global Positioning System (GPS).

The positioning unit **37** measures the position of the vehicle **12** once a second, for example. The positioning unit **37** sends data representing the measured position to the ECU **31**.

The ECU **31** receives the data sent from the positioning unit **37**. The ECU **31** associates the computed amount of salinity on the road surface with the position of the vehicle **12** at a time when the concentration sensor

36 senses the salinity concentration. The ECU **31** sends data representing the computed amount of salinity on the road surface and data representing the associated position of the vehicle **12** to the server computer **14.**

The computation of the amount of the residual salinity on the road surface may be performed by the server computer **14,** instead of the ECU **31.** In this case, the ECU **31** sends data representing the salinity concentration sensed by the concentration sensor **36,** instead of data representing the computed amount of the residual salinity on the road surface, to the server computer **14.**

The data may be sent to the server computer 14 in real-time, or may be recorded in the ECU **31** and sent all at once later.

The server computer **14** is disposed apart from the vehicle **12.** The server computer **14** communicates with the ECU **31** via a wireless communication, public telecommunication network, or others.

The server computer **14** receives the data sent from the ECU **31.** The server computer **14** determines a dangerous area in the road surface, for example. In a case that an amount of residual salinity per unit area is less than a predetermined threshold, there is a risk of the road surface freezing. So, the area is determined as the dangerous area.

For example, the server computer **14** compares the amount of residual salinity with the predetermined threshold. When the amount of residual salinity is smaller than the predetermined threshold, the server computer **14** determines that the associated position of the vehicle **12** is within the dangerous area. When the amount of residual salinity is greater than the predetermined threshold, the server computer **14** determines that the associated position of the vehicle **12** is not within the dangerous area.

The comparison of the amount of residual salinity with the predetermined threshold may be performed by the ECU **31,** instead of the server computer **14.** In this case, the ECU **31** sends data representing result of the comparison, instead of, or in addition to, the data representing the computed amount of residual salinity, to the server computer **14.** Also, the determination of the dangerous area may be performed by the ECU **31,** instead of the server computer **14.**

The liquid crystal display unit **15** displays a road map. The liquid crystal display unit **15** displays the dangerous area determined by the server computer **14** superposed on the displayed map. For example, the dangerous area is represented in red. This enables to easily grasp the dangerous area, where salt or other anti-freezing agents should be scattered. The liquid crystal display unit 15 may be disposed in a vehicle for scattering anti-freezing agents. The vehicle may be the same as the vehicle **12,** or be different from the vehicle **12.**

Anti-freezing agents may be automatically scattered. For example, the server computer 14 sends data representing the dangerous area to the vehicle for scattering anti-freezing agents. When the vehicle passes the dangerous area, the vehicle automatically scatters anti-freezing agents.

Referring to FIG. 2, a salinity measurement method includes a switch monitoring step **S1,** a sprinkling step **S2,** a concentration sensing step **S3,** a salinity computation step **S4,** a threshold comparison step **S5,** a safety determination step **S6,** a danger determination step **S7,** and a map display step **S8.**

In the salinity measurement method, the process starts from the switch monitoring step **S1.**

In the switch monitoring step **S1,** the ECU **31** monitors the state of the switch **32.** In a case that the switch **32** is off, the process repeats the switch monitoring step **S1.** In a case that the switch **32** is on, the process proceeds to the sprinkling step **S2.**

In the sprinkling step **S2,** the front nozzle **34** and the rear nozzle **35** sprinkle the water stored in the water tank **33.** Then, the process proceeds to the concentration sensing step **S3.**

In the concentration sensing step **S3,** the concentration sensor **36** senses salinity concentration of the water bounced up by the measurement wheel **22.** Then, the process proceeds to the salinity computation step **S4.**

In the salinity computation step **S4,** the ECU **31** computes an amount of residual salinity per unit area on the road surface. Then, the process proceeds to the threshold comparison step **S5.**

In the threshold comparison step **S5,** the server computer **14** compares the amount of residual salinity with a predetermined threshold. In a case that the amount of residual salinity is larger than the threshold, the process proceeds the safety determination step **S6.** In a case that the amount of residual salinity is smaller than the threshold, the process proceeds the danger determination step **S7.**

In the safety determination step **S6,** the server computer **14** determines that the position of the vehicle **12** at a time when the concentration sensor **36** senses the salinity concentration is within a safe area. Then, the process proceeds to the map display step **S8.**

In the danger determination step **S7,** the server computer **14** determines that the position of the vehicle **12** at a time when the concentration sensor **36** senses the salinity concentration is within a dangerous area. Then, the process proceeds to the map display step **S8.**

In the map display step **S8,** the liquid crystal display unit **15** displays the dangerous area on a map. Then, the process returns to the switch monitoring step **S1.**

The above-described embodiments are examples to make it easier to understand the present invention. The present invention is not limited to the examples, and includes any modified, altered, added, or removed variations, without departing from the scope of the claims attached herewith. This can be easily understood by persons skilled in the art.

REFERENCE SIGNS LIST

**10**: Salinity management system; **12**: Vehicle; **13**: salinity measurement apparatus; **14**: server computer; **15**: liquid crystal display unit; **21**: front tire; **22**: rear tire; **31**: ECU; **32**: switch; **33**: water tank; **34**: front nozzle; **35**: rear nozzle; **36**: concentration sensor; **37**: positioning unit; **S1**: switch determination; **S2**: sprinkling; **S3**: concentration sensing; **S4**: salinity computation; **S5**: threshold comparison; **S6**: safety determination; **S7**: danger determination; and **S8**: map display.


**Claims**

1. An apparatus (13) for measuring salinity on a road surface, wherein the apparatus (13) is mounted in a vehicle (12) having a wheel (22), the vehicle (12) being arranged for traveling on the road surface, the apparatus (13) comprising:

   a concentration sensor (36) disposed behind the wheel (22), and for sensing salinity concentration of water bounced up by the wheel (22);
   a front nozzle (34) disposed ahead the wheel (22), and for sprinkling water toward an area of the road surface on which the wheel (22) is to pass; and
   a rear nozzle (35) disposed between the wheel (22) and the front nozzle (34), and for sprinkling water toward the wheel (22).

2. The apparatus (13) of Claim 1, further comprising an electric control unit (31) for computing an amount of the salinity on the road surface based on the salinity concentration sensed by the concentration sensor (36) and an amount of the water sprinkled by the front nozzle (34) and the rear nozzle (35).

3. A system (10) for managing salinity on a road surface, comprising:

   the apparatus (13) of Claim 1 or 2; and
   a display (15) for displaying a dangerous area on a map, the dangerous area being determined that an amount of the salinity in the dangerous area is less than a predetermined threshold, based on the amount of the salinity measured by the apparatus (13) and a position of the vehicle (12) at a time when the concentration sensor (36) senses the salinity concentration.

4. A method for measuring salinity on a road surface, wherein a vehicle (12) having a wheel (22) travels on the road surface, the method comprising:

   sprinkling water from a front nozzle (34) disposed ahead the wheel (22) toward an area of the road surface on which the wheel (22) is to pass;
   sprinkling water from a rear nozzle (35) disposed between the wheel (22) and the front nozzle (34) toward the wheel (22); and
   sensing salinity concentration of water bounced up by the wheel (22) by a concentration sensor (36) disposed behind the wheel (22).

5. The method of Claim 4, further comprising computing an amount of the salinity on the road surface based on the salinity concentration sensed by the concentration sensor (36) and an amount of the water sprinkled by the front nozzle (34) and the rear nozzle (35).

6. The method of Claim 5, further comprising:

   measuring a position of the vehicle (12) at a time when the concentration sensor (36) senses the salinity concentration; and
   determining a dangerous area in which the amount of the salinity is less than a predetermined threshold, based on the computed amount of the salinity and the measured position.

7. The method of Claim 6, further comprising displaying the determined dangerous area on a map.


**Patentansprüche**

1. Vorrichtung (13) zum Messen des Salzgehalts auf einer Straßenoberfläche, wobei die Vorrichtung (13) in einem Fahrzeug (12) montiert ist, das ein Rad (22) aufweist, wobei das Fahrzeug (12) dazu eingerichtet ist, auf der Straßenoberfläche zu fahren, wobei die Vorrichtung (13) aufweist:

   einen hinter dem Rad (22) angeordneten Konzentrationssensor (36) zum Erfassen der Salzkonzentration von Wasser, das durch das Rad (22) aufgewirbelt wird;
   eine vor dem Rad (22) angeordnete vordere Düse (34) zum Sprühen von Wasser in Richtung zu einem Bereich der Straßenoberfläche, in den das Rad (22) sich bewegen wird; und
   eine zwischen dem Rad (22) und der vorderen Düse (34) angeordnete hintere Düse (35) zum Sprühen von Wasser in Richtung zum Rad (22).

2. Vorrichtung (13) nach Anspruch 1, ferner mit einer elektrischen Steuereinheit (31) zum Berechnen eines Salzgehalts auf der Straßenoberfläche basierend auf der durch den Konzentrationssensor (36) erfassten Salzkonzentration und einer Menge des durch die vordere Düse (34) und die hintere Düse (35) ausgesprühten Wassers.

**3.** System (10) zum Managen eines Salzgehalts auf einer Straßenoberfläche, mit:

der Vorrichtung (13) nach Anspruch 1 oder 2; und

einer Anzeige (15) zum Anzeigen eines gefährlichen Bereichs, in dem der Salzgehalt niedriger ist als ein vorgegebener Schwellenwert, auf einer Karte, wobei der gefährliche Bereich basierend auf dem durch die Vorrichtung (13) gemessenen Salzgehalt und einer Position des Fahrzeugs (12) zu einem Zeitpunkt, zu dem der Konzentrationssensor (36) die Salzkonzentration erfasst, bestimmt wird.

**4.** Verfahren zum Messen des Salzgehalts auf einer Straßenoberfläche, wobei ein Fahrzeug (12), das ein Rad (22) aufweist, auf der Straßenoberfläche fährt, wobei das Verfahren die Schritte aufweist:

Sprühen von Wasser aus einer vor dem Rad (22) angeordneten vorderen Düse (34) in Richtung zu einem Bereich der Straßenoberfläche, in den das Rad (22) sich bewegen wird;
Sprühen von Wasser aus einer zwischen dem Rad (22) und der vorderen Düse (34) angeordneten hinteren Düse (35) in Richtung zum Rad (22); und
Erfassen einer Salzkonzentration von durch das Rad (22) aufgewirbeltem Wasser durch einen hinter dem Rad (22) angeordneten Konzentrationssensor (36).

**5.** Verfahren nach Anspruch 4, ferner mit dem Berechnen eines Salzgehalts auf der Straßenoberfläche basierend auf der durch den Konzentrationssensor (36) erfassten Salzkonzentration und einer Menge des durch die vordere Düse (34) und die hintere Düse (35) ausgespritzten Wassers.

**6.** Verfahren nach Anspruch 5, ferner mit den Schritten:

Messen einer Position des Fahrzeugs (12) zu einem Zeitpunkt, zu dem der Konzentrationssensor (36) die Salzkonzentration erfasst; und
Bestimmen eines gefährlichen Bereichs, in dem der Salzgehalt niedriger ist als ein vorgegebener Schwellenwert, basierend auf dem berechneten Salzgehalt und der gemessenen Position.

**7.** Verfahren nach Anspruch 6, ferner mit dem Anzeigen des bestimmten gefährlichen Bereichs auf einer Karte.

**Revendications**

**1.** Appareil (13) pour mesurer la salinité d'une surface de route, dans lequel l'appareil (13) est monté sur un véhicule (12) ayant une roue (22), le véhicule (12) étant agencé pour rouler sur la surface de route, l'appareil (13) comprenant :

un capteur de concentration (36) disposé derrière la roue (22) et pour détecter la concentration de salinité de l'eau projetée par la roue (22) ;
une buse avant (34) disposée devant la roue (22) et pour pulvériser l'eau vers une zone de la surface de route sur laquelle la roue (22) doit passer ; et
une buse arrière (35) disposée entre la roue (22) et la buse avant (34) et pour pulvériser l'eau vers la roue (22).

**2.** Appareil (13) selon la revendication 1, comprenant en outre :

une unité de commande électrique (31) pour calculer une quantité de salinité sur la surface de route sur la base de la concentration de salinité détectée par le capteur de concentration (36) et d'une quantité d'eau pulvérisée par la buse avant (34) et la buse arrière (35).

**3.** Système (10) pour gérer la salinité sur une surface de route, comprenant :

l'appareil (13) selon la revendication 1 ou 2 ; et
un affichage (15) pour afficher une zone dangereuse sur une carte, la zone dangereuse étant déterminée par le fait qu'une quantité de salinité dans la zone dangereuse est inférieure à un seuil prédéterminé, sur la base de la quantité de salinité mesurée par l'appareil (13) et d'une position du véhicule (12) à un moment où le capteur de concentration (36) détecte la concentration de salinité.

**4.** Procédé pour mesurer la salinité sur une surface de route, dans lequel un véhicule (12) ayant une roue (22) roule sur la surface de route, le procédé comprenant les faits de :

pulvériser l'eau à partir d'une buse avant (34) disposée devant la roue (22) vers une zone de la surface de route sur laquelle la roue (22) doit passer ;
pulvériser l'eau à partir d'une buse arrière (35) disposée entre la roue (22) et la buse avant (34) vers la roue (22) ; et
détecter la concentration de salinité de l'eau projetée par la roue (22) par un capteur de concentration (36) disposé derrière la roue (22).

**5.** Procédé selon la revendication 4, comprenant en outre le fait de :

calculer une quantité de salinité sur la surface de route sur la base de la concentration de salinité détectée par le capteur de concentration (36) et d'une quantité d'eau pulvérisée par la buse avant (34) et la buse arrière (35).

**6.** Procédé selon la revendication 5, comprenant en outre les faits de :

mesurer une position du véhicule (12) à un moment où le capteur de concentration (36) détecte la concentration de salinité ; et déterminer une zone dangereuse dans laquelle la quantité de salinité est inférieure à un seuil prédéterminé, sur la base de la quantité de salinité calculée et de la position mesurée.

**7.** Procédé selon la revendication 6, comprenant en outre le fait de :
afficher la zone dangereuse déterminée sur une carte.

FIG. 1

FIG. 2

Salinity Measurement

S1: Switch Monitoring

S2: Sprinkling

S3: Concentration Sensing

S4: Salinity Computation

S5: Threshold Comparison

S6: Safety Determination

S7: Danger Determination

S8: Map Display

**EP 4 056 989 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1602913 A1